# EUROPÄISCHE PATENTSCHRIFT

(11) **EP 1 717 306 B1**
(45) Veröffentlichungstag und Bekanntmachung des Hinweises auf die Patenterteilung: **07.05.2008**
(21) Anmeldenummer: 06008246.8
(22) Anmeldetag: 21.04.2006
(51) Int. Cl.: C12M 1/113

(54) **Verfahren zur Biogaserzeugung durch Trockenfermentation sowie Biogasanlage dafür**
Process and device for generating biogas using solid state fermentation
Procédé et installation de production de biogaz par fermentation en millieu solide

(30) Priorität: 27.04.2005 DE 102005019486; 09.08.2005 DE 102005037452
(43) Veröffentlichungstag der Anmeldung: 02.11.2006
(73) Patentinhaber: Wallin, Hartmut, 27374 Visselhövede (DE)
(72) Erfinder: Wallin, Hartmut, 27374 Visselhövede (DE)
(74) Vertreter: Hansen, Jochen

(56) Entgegenhaltungen:
- DE-A1- 3 719 564
- DE-A1- 3 814 442
- DE-U1-6202004 012 74
- US-A1- 2003 059 927

## Beschreibung

Die Erfindung betrifft ein Verfahren sowie eine Anlage zur Biogaserzeugung durch Trockenfermentation mit einer im wesentlichen gasdichten Umhausung, in der ein Fermenter angeordnet ist, wobei bei dem Verfahren zu vergärender Feststoff dem Fermenter zugeführt, ggf. mit Perkolatflüssigkeit benetzt und unter Bildung von Biogas (Methan, Kohlendioxid) vergärt wird.

Biogas wird in der Land- und Abfallwirtschaft durch anaerobe Vergärung (Fermentation) von organischen Stoffen (Biomasse) unter Mitwirkung verschiedener Mikroorganismen produziert, um mit dem darin enthaltenen Methan in einem Blockheizkraftwerk Strom und Wärme zu erzeugen. Zusätzlich wird eine geruchsfreie Kompostierung der Biomasse erreicht, was in der Abfallwirtschaft Hauptzweck des Verfahrens ist. Die mikrobiellen und organchemischen Vorgänge entsprechen zu großen Teilen der Wiederkäuerverdauung. Die Vergärung geschieht in vier Phasen: Hydrolyse, Versäuerung, Acetogenese und Methanogenese. Die Phasen der Hydrolyse und Versäuerung arbeiten in anderen Bereichen hinsichtlich des PH-Wertes und der Temperatur optimal, als die Phasen Acetogenese und Methanogenese. Es gibt zweistufige Verfahren die diesem Umstand Rechnung tragen und einstufige Verfahren, die sich nach den Optimalbedingungen der Methanogenese richten, weil die anderen Phasen auch unter suboptimalen Bedingungen funktionieren. Die Methanogenese kommt dagegen bei zu niedrigen PH- Werten zum Erliegen. In der Praxis überwiegen aus Kostengründen die einstufigen Verfahren.

Das Problem der zweistufigen Verfahren sind die hohen Kosten. Bei einstufigen Verfahren entsteht ein Zeitverlust durch die suboptimalen Verhältnisse für die Hydrolyse- und Versäuerung. Zudem ist das Verfahren anfällig gegen "Fütterungsfehler" die eine Übersäuerung im Fermenter bewirken und die Methanogenese zum Erliegen bringen und im schlimmsten Fall eine Entsorgung des gesamten Substrates erfordern.

Daneben gibt es Pfropfenströmungsverfahren, bei denen die Biomasse durch Verdrängung bei Zugabe von frischer Biomasse oder durch Sog bei Entnahme im Fermenter in eine Richtung bewegt wird. Dadurch wird eine annähernde Trennung der vier Phasen der Vergärung erreicht.

Die Biogaserzeugung geschieht mit Hilfe verschiedener Verfahren der Flüssigfermentation (pump-und rührfähige flüssige Biomasse oder durch Anmischung mit wässriger Flüssigkeit pump- und rührfähig gemachte Biomasse) oder durch "Trockenfermentation".

Die Flüssigfermentation ist bei Betrieb ohne Gülle problematisch, weil die meisten anderen organischen Stoffe mit hohen Energieaufwand und Wasserbedarf mit wässriger Flüssigkeit so gemischt werden müssen, dass sie pump- und rührfähig sind. Ein weiteres Problem ist, dass Gräserarten frisch oder silert, z.B. Grassilage vom Grünland, Ganzpflanzensilage von Getreidearten (außer Mais) nur in begrenzten Umfang einsetzbar sind, weil sie bei gleichem Trockensubstanzgehalt wie andere Biomasse der Gesamtmischung schwerer zu rühren sind, und dazu neigen Schwimmschichten im Behälter zu bilden. Die Verfahren sind anfällig für Verunreinigungen der zugeführten Biomasse. Durch das ständige Rühren im Fermenter wird die frische Masse mit der bereits vergorenen vermischt, so das beim Ausbringen frische Biomasse der Biogaserzeugung verloren geht.

Bei der Trockenfermentation wird stapelfähige Biomasse (Trockensubstanzgehalt: 20% - 50%) zerkleinert und durchmischt dem Fermenter zugeführt. Das für die bakterielle Aktivität notwendige Wasser ist zum einen in der Biomasse, wird zum anderen in der Praxis durch Versprühen über dem Gärstapel der Biomasse zugeführt. Das nach unten durchgesickerte Wasser wird in einem Tank aufgefangen, aus dem dann wieder Flüssigkeit zum Versprühen gefördert werden kann. Der Vorgang wird Perkolation genannt.

Die Flüssigfermentation ist praxisbewährt wirtschaftlich. Die Trockenfermentation ist im landwirtschaftlichen Bereich im Pilotstadium, in der Abfallwirtschaft praxisbewährt, weil hier die geruchsfreie Kompostierung Hauptzweck ist.

Eine Möglichkeit der Trockenfermentation ist das diskontinuierliche Verfahren in "Garagen".

Die eingangs genannten Verfahren und Anlagen zur Biogaserzeugung durch Trockenfermentation für den diskontinuierlichen, sog. Batch-Betrieb sind beispielsweise aus der EP 0 934 998 oder DE 100 50 623 A1 bekannt. Dabei wird der Fermenter mit frischem Material gefüllt und luftdicht verschlossen. Während einer Faulzeit von einigen Wochen baut sich die vergärbare Substanz unter Abgabe von Biogas ab. Anschließend wird der Fermenter geleert und wieder mit neuem Frischmaterial befüllt. Da beim Batch-Betrieb die Gasproduktion und die Gaszusammensetzung nicht konstant sind, müssen für einen wirtschaftlichen Betrieb eines nachgeschalteten Blockheizkraftwerks oder dergleichen mehrere Batch-Einheiten parallel und phasenverschoben betrieben werden. Ferner ist das Befüllen und Entleeren der Fermenter arbeitsaufwendig.

Insgesamt sorgen beim diskontinuierlichen Garagenverfahren verschiedene Probleme für einen niedrigen Gasertrag je Tag und zugeführter organischer Subtstanz:
- Die Perkolation ist nur dann erfolgreich, wenn die Biomasse grob strukuriert ist. Dadurch ist die Oberfläche der Biomasse kleiner und bietet so den Mikroorganismen weniger Angriffsfläche. Die Perkolation erfolgt unkontrolliert, und wird durch die zufällige Struktur der Schüttung bestimmt. Dadurch gibt es unterschiedliche Bereiche der Perkolatwasserversorgung und daraus resultierend der Aktivität der Mikroorganismen, wodurch einer längere Verweilzeit in der Garage geplant werden muss.
- Die Vermischung von frischer Biomasse und vergorener Biomasse vor der Beschickung kostet Zeit, Energie und Fermenterraum.
- Da die Garage regelmäßig geöffnet wird kommt es zu Emissionen von Biogas und es entsteht eine explosive Methan-Luftmischung, die ein beherrschbares aber vorhandens Risiko bildet.
- Die notwendige Heizung ist in großen Behältern durch die Dämmwirkung der Biomasse weitgehend wirkungslos. Dadurch fällt die Temperatur in Praxisanlagen innerhalb von 3 Wochen von 55 - 60°C auf ca. 27°C. Bei Temperaturen unter 30° ist die Produktion von Biogas durch die Mikroorganismen eingeschränkt.
- Durch die Vorerhitzung der Biomasse durch aerobe Kompostierung geht organisches Material verloren und es entsteht zusätzlicher Aufwand.
- Die Gaserträge in der Garage schwanken stark. Da die Anzahl der Volllaststunden des Blockheizkraftwerkes wegen hoher Abschreibungskosten entscheidend für die Wirtschaftlichkeit des Verfahrens ist, müssen mehrere Garagen gebaut werden.
- Das gebildete Biogas löst sich im Gärstapel nicht so gut von der Biomasse. Deshalb entsteht beim Austrag ein Methan-Luftgemisch an der Atmosphäre.
- Die durch die Biogasproduktion (Entzug von Masse) bedingte tägliche Volumenverkleinerung des Stapels kann nicht ausgeglichen werden.
   Somit wurde auch für die Vergärung von Feststoffen versucht, kontinuierliche Verfahren zu entwickeln.
   Gemäß FAT-Berichte Nr. 451, 1994 der Eidgenössischen Forschungsanstalt für Agrarwirtschaft und Landtechnik in Tänikon, Schweiz ist daher eine kontinuierlich betriebene Biogasanlage zur Vergärung von strohreichem Mist beschrieben worden, bei der der Fermenter mit einer Kolbenpresse (Maulwurf) auf seiner Oberfläche mit Mist beschickt wird, welcher im Durchlaufverfahren vergoren wird. Der Austrag des vergorenen Materials erfolgt mit Hilfe eines Kratzbodens auf der Unterseite des Fermenterraums mit einer darunter angeordneten Austragschnecke. Im Beschickungsrohr der Kolbenpresse wird der zugeführte zu vergärende Mist auf die erforderliche Gärtemperatur aufgewärmt. Im Kopfbereich des Fermenters erfolgt die Gasentnahme.
   Nachteilig an dieser Pilotanlage ist, dass eine Erweiterung auf größere Dimensionen nicht ohne weiteres möglich ist, da die Dämmwirkung des Stapels bei der dort verwirklichten außen liegenden Vorerhitzung wiederum zu einer zu niedrigen Temperatur innerhalb des Gärstapels führen kann. Ferner ist das im Gärstapel entstehende Biogas weiterhin in dem kompakt übereinander liegenden Material eingebunden, womit ein Teil des Biogases erst nach Verlassen des Fermenterraums aus dem vergorenen Material austreten wird.
   Aufgabe der Erfindung ist es daher, ausgehend von der kontinuierlich betriebenen Biogasanlage gemäß FAT-Berichte Nr. 451 ein quasikontinuierliches Verfahren zur Biogaserzeugung bzw. eine Anlage dafür anzugeben, mit dem bzw. mit der ein verbesserter Methanertrag auch bei einer großdimensionierten Anlage ermöglicht wird.
   Gelöst wird diese Aufgabe mit einem Verfahren gemäß Anspruch 1 und einer Anlage gemäß Anspruch 8.
   Mit einem Durchmischen und Erwärmen der zugeführten zu vergärenden Feststoffe in einer Vorstufe auf einer im wesentlichen horizontalen Förderbahn unterhalb des Gärstapels innerhalb der gasdichten Umhausung, wird ein gesondertes Durchmischen der zuzuführenden Feststoffe vor der Einleitung in die Anlage entbehrlich. Durch die vom Gärstapel auf die zugeführten zu vergärenden Feststoffe niedertropfende Perkolatflüssigkeit und/oder niederfallende kleinere Fraktionen aus dem Gärstapel werden die zu vergärenden Feststoffe bereits intensiv mit den für den Gärprozess erforderlichen Mikroorganismen in Kontakt gebracht und auf die für den Gärprozess erforderliche Temperatur erwärmt.
   Durch das Verteilen der erwärmten und durchmischten Feststoffe aus der Vorstufe auf dem im Fermenter befindlichen Gärstapel wird eine neue Schicht zu vergärender Feststoffe auf den Gärstapel aufgebracht, die durch die Erwärmung, Durchmischung und Infektion mit den erforderlichen Mirkoorganismen für den Gärprozess optimal vorbereitet ist. Da die vergärten Feststoffe auf der Unterseite des im Fermenter befindlichen Gärstapels ausgetragen werden, bewegen sich die zu vergärenden Feststoffe schichtenweise von oben nach unten durch den Fermenter, womit sich jeweils optimale Umgebungsbedingungen für Hydrolyse, Versäuerung, Acetogenese und Methangenese in den übereinanderliegenden Schichten ausbilden können. Entsprechend soll nachfolgend auch vom sog. Schichtenströmungsverfahren gesprochen werden.
   Wenn die Förderbahn einen Förderkreislauf bildet, der von den zu vergärenden Feststoffen wenigstens einmal durchlaufen wird, bevor die dann durchmischten Feststoffe zur Verteilung auf dem Gärstapel emporgefördert werden, können die zugeführten zu vergärenden Feststoffe je nach Material und Vormischungsverhältnis bedarfsweise auch länger/intensiver in der Vorstufe bearbeitet werden.
   Vorrichtungsgemäß wird dies dadurch erreicht, dass das Horizontalfördermittel als Kreisförderer ausgebildet ist, insbesondere in Form eines Kettenförderers. Ein Kettenförderer erlaubt eine robuste Art für einen Horizontaltransport, bei dem die eingebrachten Feststoffe auf dem Boden aufliegen und somit auch intensiv erwärmt werden. Die an der umlaufenden Kette befestigten Räumschienen nehmen dabei immer nur einen Teil der eingebrachten Feststoffe mit, so dass die erwünschte Durchmischung erfolgt.
   Wenn das Beheizungsmittel eine Fußbodenheizung und/oder Wandheizung ist, wird das auf dem Boden aufliegende bzw. an den Seiten- und Zwischenwänden anliegende Feststoffmaterial wirksam erwärmt.
   Dadurch, dass zum Austragen der vergorenen Feststoffe die Zufuhr von zu vergärenden Feststoffen gestoppt wird und vergorene Feststoffe aus dem Gärstapel auf die darunterliegende Förderbahn abgegeben und von dort ausgetragen werden, wird auch eine schichtenweise Entleerung des Fermenters angegeben. Wenn die auszugebenden vergorenen Feststoffe den Förderkreislauf der Förderbahn wenigstens einmal durchlaufen, werden die aus dem Gärstapel in seinem Bodenbereich abgegebenen vergorenen Feststoffe in den unterflurigen Raum durch die Horizontalfördermittel, insbesondere Kettenförderer intensiv aufgelockert und letztmalig erwärmt, um darin eingeschlossenes Biogas freizugeben und letzte Biogas bildende Aktivitäten auszulösen.
   Um ein im wesentlichen gleichmäßiges Schichtenströmungsverfahren erzeugen zu können, bestehen die Öffnungen im Boden des Fermenters bevorzugt aus Spalten bzw. sind aus einem Spaltenboden, wie er für die Viehhaltung bekannt ist, gebildet. Wenn die Spalten verschließbar ausgebildet sind, kann das Abtropfen von Perkolatflüssigkeit und von kleineren Fraktionen aus dem Gärstapel reguliert werden.
   Um beim Entleerungsvorgang von vergärten Feststoffen einen intensiven und möglichst gleichmäßigen (schichtweisen) Materialaustrag zu gewährleisten, liegt ein bevorzugt hydraulisch hin- und herbewegbarer Kratzboden mit Verschlussblechen auf dem Spaltenboden auf.
   In weiterer Ausgestaltung des Verfahrens wird ein interner Kreislauf vorgeschlagen, bei dem teilweise vergorene Feststoffe auf die Förderbahn abgegeben, dort mit frisch zugeführten, zu vergärenden Feststoffen gemischt und wieder auf dem im Fermenter befindlichen Gärstapel verteilt werden. Die bereits einmal durch den Gärstapel "nach unten gewanderte" Substratschicht wird somit unter Hinzufügung von frisch zugeführten, zu vergärenden Feststoffen zum Ausgleich des durch die laufende Biogaserzeugung verbrauchten Materials im unterflurigen Raum durchmischt, wiederum erwärmt und als neue Schicht oben auf den Gärstapel abgelegt. Während der Durchmischung erfolgt wiederum eine Auflockerung, so dass eingeschlossenes Biogas freigegeben wird und das neu zugeführte zu vergärende Feststoffmaterial intensiv mit den Mikroorganismen in Kontakt gerät.
   Bevorzugt wird somit bei einem vollständig befüllten Fermenter nach dem internen Kreislauf zunächst eine Teilmenge des Gärstapels über die Förderbahn ausgegeben, dann zu vergärende Feststoffe über die Förderbahn zugeführt und auf dem Gärstapel verteilt und anschließend wieder ein interner Kreislauf durchgeführt und dann wiederum ein Ausgabe- und Zuführvorgang durchgeführt. Diese Verfahrensfolge wiederholt sich dann ständig, so dass ein quasikontinuierlicher Betrieb sichergestellt ist.
   Dadurch, dass Verteilungsmittel zur im wesentlichen gleichmäßigen schichtweisen Ablage von zugeführten Feststoffen auf dem Gärstapel vorgesehen sind, insbesondere in Form von hin- und herbewegbaren Balken, kann eine schichtweise Befüllung des Fermenters mit einer im wesentlichen gleichmäßigen Schichtdicke erreicht werden.
   Wenn zum Austrag von vergorenen Feststoffen eine bedarfsweise antreibbare Förderschnecke im Bereich des unterflurigen Raumes, bevorzugt unterhalb des Horizontalfördermittels, angeordnet ist, können je nach Bedarf vergorene Feststoffe aus der Anlage ausgetragen werden, wobei bei nicht angetriebener Förderschnecke die Feststoffe zunächst noch im unterflurigen Raum mittels des Horizontalfördermittels auf einen geschlossenen Förderweg befördert werden. Dabei werden die vergorenen Feststoffe nochmals durchmischt und insofern auch aufgelockert, so dass in den Feststoffen eingeschlossene Biogase vor dem endgültigen Austrag aus der Anlage freigegeben werden.
   Wenn innerhalb der gasdichten Umhausung der Perkolatflüssigkeitskreislauf mit Vorratstank, Förderleitungen, Pumpen, Sprühköpfen und Auffangsumpf angeordnet sind, wobei der Vorratstank eine Beheizung aufweist und die Sprühköpfe oberhalb des Gärstapels angeordnet sind, sind alle für die Perkolatzirkulation erforderlichen Vorrichtungen innerhalb der gasdichten Umhausung angeordnet. Ferner kann eine Perkolatflüssigkeitsleitung vom Vorratstank zu einer oder mehreren Beregnungsstellen im Bereich der Förderbahn zur Perkolat-Beregnung von darauf aufliegenden zu vergärenden Feststoffen vorgesehen werden. Alternativ kann der unterflurige Raum auch von einer perforierten Perkolatleitung im wesentlichen ganzflächig besprüht werden. Um diese bedarfsweise zusätzliche Perkolation in Abhängigkeit der jeweiligen Verfahrensabfolge steuern zu können, sind entsprechend steuerbare Ventile und ggf. Pumpen zur Förderung der Perkolatflüssigkeit vorzusehen.
   Wenn die vorgenannten Bauteile alle innerhalb der gasdichten Umhausung angeordnet sind, sind ergänzende Bauten und Dichtigkeits- und Wärmedämmprobleme an den Zirkulationsleitungen sowie gesonderte Beheizungsanlagen somit nicht mehr nötig. Selbstverständlich kann im Perkolatvorratstank eine bedarfsweise Beheizung vorgesehen werden, um das auf die jeweils neu ausgebrachte Schicht aufzubringende Perkolat auf eine optimale Prozesstemperatur vorzutemperieren. Ferner kann bei dieser Konstellation das im Perkolatvorratstank entstehende Biogas dem im Fermenter erzeugten Biogas unmittelbar durch freie Öffnungen am Kopf des Vorratstankes zugeführt werden. Aufwändige Gasüberleitleitungen von externen Perkolatflüssigkeitstank können entfallen.
   Dabei wird die Perkolatflüssigkeit über dem Gärstapel gleichmäßig versprüht, sinkt im Gärstapel nach unten, tropft auf die Förderbahn und wird darunter zur erneuten Verwendung gesammelt. Ergänzend kann Perkolatflüssigkeit auch direkt auf das auf der Förderbahn befindliche Material, insbesondere neu zugeführte, zu vergärende Feststoffe, aufgesprüht werden. Dabei ist es denkbar, dass Beregnungsrohrleitungen in Form von fein perforierten Rohrleitungen im unterflurigen Raum zum Benetzen der auf der Förderbahn aufliegenden zu vergärenden Feststoffe vorgesehen sind. Alternativ kann auch an einer oder mehreren Stellen der kreisförmig geführten Förderbahn eine örtlich begrenzte Beregnung mit Perkolatflüssigkeit in Art einer Dusche oder dergleichen ausgeführt sein.
   Bevorzugt wird das Biogas beim Auffangen schwerkraftbedingt im wesentlichen in eine Methanfraktion und eine Fraktion der übrigen Bestandteile, insbesondere Kohlendioxid getrennt. Dies wird vorrichtungsgemäß dadurch erreicht, dass die Umhausung ein Gasspeichervolumen im wesentlichen oberhalb des Fermenters aufweist, wobei im oberen Bereich des Gasspeichervolumens in der Umhausung eine erste Gasentnahmeleitung und im Bodenbereich der Umhausung eine zweite Gasentnahmeleitung angeordnet sind.
   Bevorzugt beträgt das für den Gärstapel nutzbare Volumen des Fermenters 50 bis 200 m³, bevorzugt ca. 100 m³, das Volumen des unterflurigen Raumes 8 bis 32 m³, bevorzugt ca. 16 m³, und das Gasspeichervolumen 50 bis 200 m³, bevorzugt ca. 100 m³.
   Erfindungsgemäß wird somit eine Anlage und ein Verfahren zur Biogaserzeugung durch Trockenfermentation angegeben, bei dem in einer Vorstufe innerhalb der Umhausung des Fermenters eine intensive Erwärmung der frisch zugeführten Biomasse bzw. der rezirkulierten Biomasse erfolgt. Dabei wird die Erwärmung bevorzugt durch Oberflächenkontakt auf der Fußbodenheizung und/oder den Wandheizungen der Wände bzw. Zwischenwände erreicht. Da diese Erwärmung innerhalb der Gesamtumhausung der Anlage durchgeführt wird, wird zum größten Teil auch dissipative Wärmeenergie dem Gesamtprozess in dem darüberliegenden Gärstapel zugeführt. Zusätzlich kann der Gärstapel über Wandheizungen selbst erwärmt werden. Die intensive Erwärmung in dem unterflurigen Bereich wird auch beim internen Kreislauf, also der Rezirkulierung einer bereits einmal vergärten Schicht aus dem Gärstapel ausgenutzt, um optimal vorerwärmte Biomasse wieder erneut auf den Gärstapel aufzubringen. Eine unerwünscht starke Abkühlung im Gärstapel wird somit verhindert. Zudem wird durch die schichtweise Ablage der zu vergärenden Feststoffe im sog. Schichtenströmungsverfahren für den jeweiligen Prozessabschnitt optimale Milieubedingungen geschaffen, so dass die Verweilzeit der Biomasse im Fermenter gegenüber herkömmlichen Verfahren erheblich verkürzt ist. Entsprechend ist damit auch nur eine geringere Vorerwärmung zum Ausgleich der zeitbedingten Temperaturverluste nötig.
   Ferner wird die Infektion der frisch zugeführten, zu vergärenden Feststoffe in dem erfindungsgemäß vorgesehenen unterflurigen Raum sehr wirksam durch eine breitflächig und aufgelockerte Verteilung des zugeführten Materials erreicht. Das Material wird durch den Kettenförderer langsam horizontal transportiert, mit den für den Gärprozess erforderlichen Mikroorganismen durch vom Gärstapel abtropfende Perkolatflüssigkeit und/oder kleineren Fraktionen vergärter Feststoffe versorgt und durch den Horizontaltransport intensiv durchmischt. Dabei ist als weiterer Vorteil herauszustellen, dass das aus dem Gärstapel heraustropfende Perkolat eine höhere Methanbakteriendichte aufweist, als das im Vorratstank vorgehaltene Perkolat, da im Tank viele Methanbakterien aus Nahrungsmangel absterben. Zudem erfolgt die Verteilung der Biomasse auf dem Gärstapel breitflächig und auflockernd, so dass die Perkolation hier ebenfalls hochwirksam ist.
   Das Problem der Bindung des Methangases innerhalb des vergorenen Substrats wird dadurch gelöst, dass beim Horizontaltransport im unterflurigen Raum unterhalb des Fermenters das Gas von der vergorenen Biomasse durch den Auflockerungsvorgang getrennt wird und erst danach die vergorenen Feststoffe ausgetragen werden.
   Durch die schichtweise Befüllung und die Perkolation im unterflurigen Raum ist ein so großer Kontakt der Frischmasse mit dem mikrobiologisch aktiven Gärstapel gegeben, dass die Gärung in der frischen Biomasse unverzüglich angestoßen wird. Wenn schnell abbaubare Biomasse eingebracht wird, die zu einer Übersäuerung des Prozesses führen kann, wird diese unterflurig mit dem aktiven Gärsubstrat gemischt, in dem die schnell abbaubare Biomasse vor Durchführen des internen Kreislaufs in dem unterflurigen Raum verteilt wird.
   Die Kosten für die Vormischung von Komponenten der Frischmasse können gesenkt werden, da die verschiedenen Beförderungen der zugeführten Feststoffe innerhalb der Anlage zu einer optimalen Durchmischung führen. Ggf. ist bei Eintrag verschiedener Komponenten eine grobe Vormischung vorteilhaft. Ingesamt sollte die Biomasse durch Schneidetechnik so weit kleinstrukturiert werden, dass eine große Oberfläche zum Angriff der Mikroorganismen entsteht, wobei die Gefahr einer Klumpenbildung durch die quasikontinuierliche Bewegung im Fermenter nicht gegeben ist bzw. etwaige Klumpen schnell aufgelöst werden.
   Die Erfindung erreicht folgende Vorteile:
   - Keine Durchmischung mit vergorener Biomasse notwendig.
   - Keine Erwärmung durch aerobe Vorverrottung notwendig.
   - Direkte Erwärmung der Biomasse durch die vorhandene Fußbodenheizung.
   - Hygienisierung bis 70 Grad Celsius durch die Fußbodenheizung möglich.
      Dies ist bei der Vergärung von bestimmten Bioabfällen vorgeschrieben.
   - Durch die Durchführung des internen Kreislaufs ist eine nochmalige Erwärmung und Auflockerung der Biomasse möglich.
   - Vor dem internen Kreislauf kann schnell angreifbare Biomasse mit hohem Ertragspotential zur Mischung in den unterflurigen Raum eingebracht werden.
   - Die annähernd schichtenweise Beschickung und Austragung der Biomasse ermöglicht es, den Teil der Biomasse auszutragen, der soweit vergoren ist, dass der Gasertrag der weiteren Vergärung unwirtschaftlich wäre.
   - Die Volumenverkleinerung der Biomasse durch Fermentation kann in der quasikontinuierlichen Beschickung kurzfristig ausgeglichen werden.
   - Bis zu sechs verschiedene Transport- und Verteilungstechniken bewirken jeweils ein Durchmischen der Biomasse.
   - Weiterhin können Ausbringen und Einbringen Zug um Zug durchgeführt werden. Dadurch wird eine chemische und mikrobielle Abpufferung der chemischen und mikrobiellen Vorgänge während des Einbringens gefördert, die eine Übersäuerung verhindern.
   - Bei der Verteilung über dem Gärstapel ist die Biomasse gut erwärmt und perkoliert. Darum wird die Biomasse von den Mikrorganismen aus dem Gärstapel schnell angegriffen.
      Durch das quasikontinuierliche Verfahren ergeben sich weitere Vorteile:

   - Keine umweltschädlichen Methanverluste durch regelmäßiges Öffnen des Behälters.
   - Kein Explosionsrisiko durch Methan-Luft Gemisch während der regelmäßigen Austragung des Behälters.
   Nachfolgend werden zwei Ausführungsbeispiele der Erfindung anhand der beiliegenden Figuren detailliert beschrieben.

Darin zeigt:
- Fig. 1: ein Ausführungsbeispiel einer Biogasanlage in teils geschnittener Draufsicht,
- Fig. 2: die Biogasanlage in einem in Fig. 1 markierten Längsschnitt,
- Fig. 3: die Biogasanlage in einem in Fig. 1 markierten, nahe einer Stirnseite angeordneten Querschnitt,

- Fig. 4: die Biogasanlage in einem in Fig. 1 markierten, mittig angeordneten Querschnitt und
- Fig. 5: ein alternatives Ausführungsbeispiel der vorgenannten Biogasanlage mit einem Schub-Zugboden.

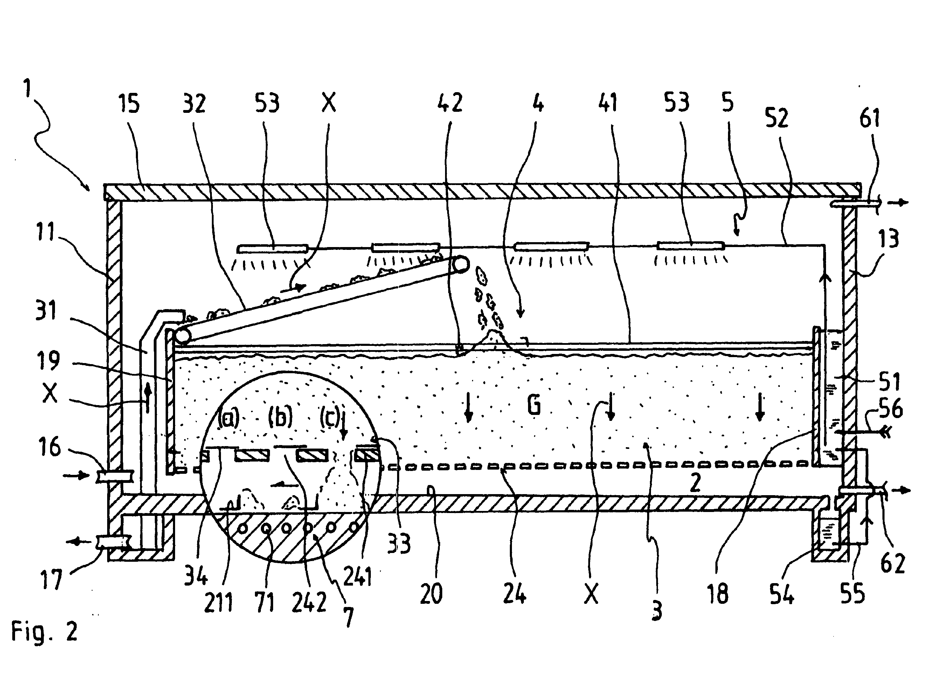

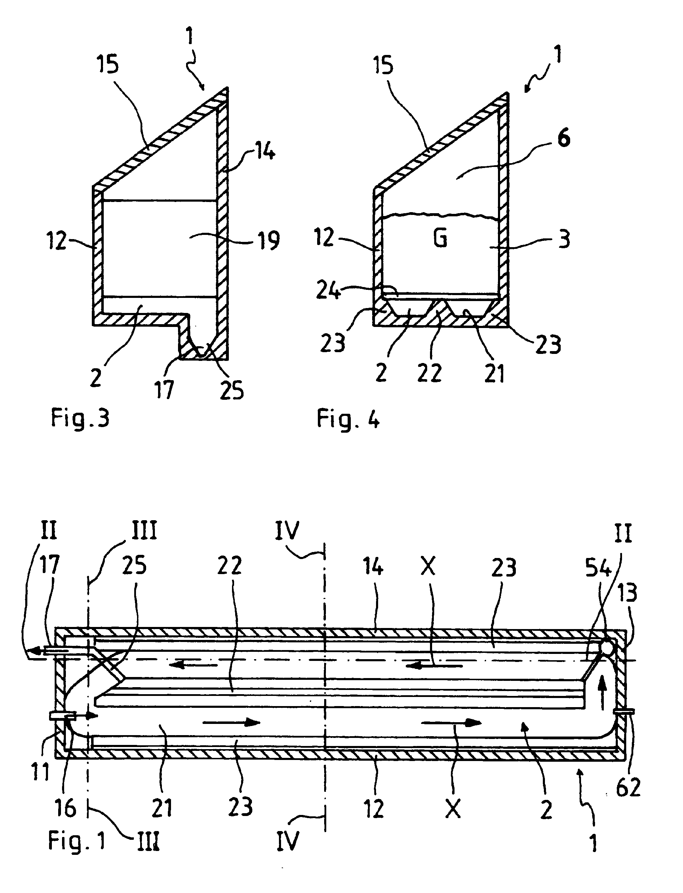

In Fig. 1 ist in teils geschnittener Draufsicht eine Anlage zur Biogaserzeugung durch Trockenfermentation abgebildet. Die Anlage weist eine Umhausung 1 auf, in der ein Fermenter 3 (s. Fig. 2) über einem unterflurigen Raum 2 angeordnet ist. Die Umhausung 1 bildet ein langgestrecktes Gebäude mit einer kurzen Stirnwand 11, daran anschließenden langgestreckten zwei Seitenwänden 12, 14 und einer die beiden Seitenwände 12, 14 verbindenden kurzen Rückwand 13. Wie in den Querschnitten der Fig. 3 und 4 ersichtlich, ist die in Fig. 1 oben dargestellte Seitenwand 14 erheblich höher ausgebildet, so dass die Umhausung 1 mit einem luftdicht abgeschlossenen Pultdach 15 gedeckt ist.

In Fig. 1 ist der unterflurige Raum 2 in Draufsicht dargestellt. Der unterflurige Raum 2 weist ein Horizontalfördermittel 21 auf, das als kreisförmig angeordneter Kettenförderer ausgebildet ist. Im unterflurigen Raum 2 ist ein leicht geneigter Fußboden 20 unter dem Horizontalfördermittel 21 ausgebildet. Im Fußboden 20 sind als Beheizungsmittel 7 Heizrohre 71 als Fußbodenheizung integriert, wie dies in der Vergrößerung in Fig. 2 ersichtlich ist. Der Kettenförderer 21 ist um einen Betonsockel 22, der parallel zu den Seitenwänden 12, 14 mittig in der Umhausung 1 angeordnet ist, im Kreis herumgeführt. Der Betonsockel 22 hat trapezförmigen Querschnitt, wie aus Fig. 4 ersichtlich. Ebenso sind an den Außenrändern zwischen dem Kettenförderer 21 und den Seitenwänden 12, 14 dreiecksförmige Betonsockel 23 angeordnet, so dass in den unterflurigen Raum 2 eingebrachte Feststoffe durch die von den Betonsockeln 22, 23 ausgebildeten Schrägen zum Kettenförderer 21 geführt werden. Ferner bilden die Oberseiten dieser Betonsockel 22, 23 die Auflage für einen Spaltenboden 24, wie er in Fig. 2 und 4 dargestellt ist.

In der Stirnwand 11 ist kurz oberhalb des Kettenförderers 21 eine Zuführöffnung 16 zum Zuführen von zu vergärenden Feststoffen sowie eine Austragöffnung 17 vorgesehen. Die Austragöffnung 17 ist, wie in Fig. 3 im Querschnitt ersichtlich, unterhalb der Ebene des Horizontalfördermittels 21 (Kettenförderer) angeordnet. Ferner ist im Bereich der Austragöffnung 17 unter dem Horizontalfördermittel 21 eine Förderschnecke 25 zum seitlichen Austrag von auf dem Horizontalfördermittel 21 geförderten Feststoffen angeordnet. An die Förderschnecke 25 schließt sich wahlweise ein Vertikalförderer 31 oder eine nicht näher dargestellte Austragschnecke an, die bereits vergorene Feststoffe durch die Austragöffnung 17 aus der Umhausung 1 hinausbefördert.

Der Vertikalförderer 31 fördert in der Vorstufe im unterflurigen Raum 2 vorbehandelte Feststoffe bis oberhalb des Fermenterraums 3 zu einem Bandförderer 32, der die Feststoffe etwa über der Mitte des Fermenters 3 abwirft und somit im Fermenter einen Gärstapel G aufbaut. Auf Höhe der Sollbefüllhöhe des Fermenters 3 ist ein Verteilungsmittel 4 in Form von zwei über dem Gärstapel G mittels Stahlseilzügen 41 hin- und herbewegbaren Balken 42 vorgesehen. Mit diesem Verteilungsmittel 4 wird ein Gärstapel G schichtenweise im Fermenter 3 auf dem Spaltenboden 24 aufgebaut. Auf dem Spaltenboden 24 ist ein Kratzboden 33 mit Verschlussblechen 34 angeordnet, der bevorzugt in einem auf dem Spaltenboden 24 aufliegenden Stahlrahmen eingefasst ist und von nicht dargestellten Hydraulikzylindern um einen Stellweg im wesentlichen entsprechend der Spaltenbreite des Spaltenbodens 24 in Längsrichtung hin- und herbewegbar ausgebildet ist. In der vergrößerten Darstellung in Fig. 2 sind einzelne Balken 241 des Spaltenbodens 24 im Querschnitt dargestellt, wobei die zwischen zwei einandergrenzenden Balken 241 ausgebildeten Spalten 242 je nach Stellung des mit den Verschlussblechen 34 ausgebildeten Kratzbodens 33 entweder ganz geschlossen (a), einen kleinen Spalt (ca. 1 cm) geöffnet (b) oder vollständig geöffnet (ca. 16,5 cm) (c) sind.

Hinter einer vor der Rückwand 13 auf den Betonsockeln 22, 23 angeordneten Zwischenwand 18 ist ein Tank 51 für Perkolatflüssigkeit angeordnet. Vom Perkolattank 51 führt eine Förderleitung 52 mit nicht dargestellter Pumpe zu über den Gärstapel G des Fermenters 3 angeordneten Sprühköpfen 53. Die von dort auf den Gärstapel G aufgesprühte Perkolatflüssigkeit gelangt nach Einwirkung auf den Gärstapel und ggf. die im unterflurigen Raum 2 befindlichen Feststoffe über den leicht geneigten Fußboden 20 in einen Auffangsumpf 54, vom dem eine Förderleitung 55 mit nicht dargestellter Pumpe eine Rückführung der aufgefangenen Perkolatflüssigkeit in den Tank 51 erlaubt. Ferner ist eine Zuführleitung 56 zum Auffüllen des Perkolattanks 51 mit von außen zugeführter neuer Perkolatflüssigkeit vorgesehen.

Zur Gasentnahme ist am höchsten Punkt des Pultdaches 1 5 nahe der hohen Seitenwand 14 eine erste Gasentnahmeleitung 61 und oberhalb des Fußbodens 20 im unterflurigen Raum 2 eine zweite Gasentnahmeleitung 62 vorgesehen. Der gesamte Hohlraum innerhalb der gasdicht ausgebildeten Umhausung 1 bildet ein Gasspeichervolumen 6, das sich somit vom Boden 20 des unterflurigen Raums 2 bis zum höchsten Punkt unter dem Pultdach 15 erstreckt.

Bei einer bevorzugten Dimensionierung der Biogasanlage beträgt die Länge der Behausung 1 ca. 15 m, die Breite ca. 3,2 m, die niedrigere Seitenwand 12 ca. 3,5 m Höhe und die höhere Seitenwand 14 ca. 6 m Höhe. Der Spaltenboden 24 besteht aus in der Viehwirtschaft bekannten Balken 241 mit einer Breite von 17,5 cm, die in einem Spaltenabstand 242 von 16,5 cm angeordnet sind. Die Balken sind an den Seitenwänden 12 und 14 auf jeweils 5 cm breit ausgebildeten Auflageflächen der dreiecksförmigen Betonsockel 23 und dem oben ca. 10 cm breiten trapezförmigen Betonsockel 22 aufgelagert. Der Kettenförderer 21 überdeckt ca. eine Breite von 80 cm auf dem Fußboden 20. Eine weitere Zwischenwand 19 ist in ca. 1 m Abstand von der Stirnwand 11 aufgebaut und grenzt den Fermenterraum 3 so ein, dass ein schmaler Bereich innerhalb der Behausung 1 für den Vertikalförderer 31, Zuführöffnung 16 und Austragöffnung 17 auch für etwaige Wartungsarbeiten frei bleibt.

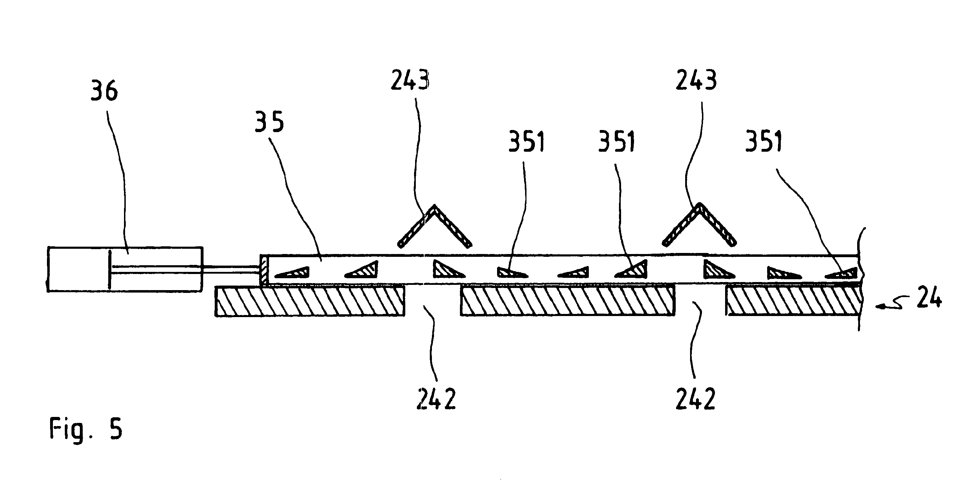

In Fig. 5 ist eine alternative Ausführungsform der Biogasanlage in einer Detailansicht ähnlich der Detailansicht in der Fig. 2 in einem Längsschnitt dargestellt. In der Fig. 5 ist der mit Spalten versehene Boden 24 über dem unterflurigen Raum 2 aus breiteren Bodenelementen, beispielsweise einer Breite von ca. 2 m und dazwischen liegenden Spaltenbereich, beispielsweise 20 bis 30 cm, ausgeführt. Funktionsgleiche Bauteile sind bei diesem Ausführungsbeispiel mit gleichen Bezugszeichen wie im ersten Ausführungsbeispiel gemäß der Fig. 1 bis 4 bezeichnet.

Im Gegensatz zum ersten Ausführungsbeispiel sind in der zweiten Ausführungsform die Spalten 242 nicht verschließbar ausgebildet. Vielmehr ist über den Spalten 242 ein dachförmiger Balken 243 beabstandet zum Boden 24 im Fermenter 3 ortsfest angeordnet, so dass bei der im Gärstapel G entstehenden Transportrichtung gemäß Pfeil X eine Aufspaltung des Gärstapels G an dem dachförmigen Balken 243 erfolgt.

Auf dem mit Spalten 242 versehenen Boden 24 ist ein Schub-Zugrahmen 35 mit leiterförmigen Schubelementen 351 angeordnet. Die Schubelemente 351 weisen dreiecksförmige Querschnittsfläche auf, wobei eine steil stehende Flanke und eine schräg auflaufende Auflauffläche ausgebildet sind. Die steil aufstehende Fläche dient als Schubriegel, wohingegen die Auflaufschräge ein Unterfahren des Gärstapels ermöglicht. Mit der steil aufstehenden Schubfläche schiebt das Schubelement 351, das im Schub-Zugrahmen 35 fest in Leiterform verankert ist, bei Betätigung des Schub-Zugrahmens 35 mittels geeigneter Hydraulikzylinder 36 ein definiertes Volumen des Gärstapels G Richtung Spalt 242. Bei der Rückbewegung rutscht die Auflaufschräge des Schubelementes 341 unter den Gärstapel, diesen im wesentlichen nicht beeinflussend, durch. Bei dieser Bewegung fördern andere Schubelemente 351, die ihre Schubfläche zu dieser Bewegungsrichtung normal orientiert haben, ebenfalls eine definierte Menge des Gärstapels G zu einem Spalt 242. Mit der erfindungsgemäß in diesem Ausführungsbeispiel vorgesehenen Schub-Zugrahmen 35 wird ein besonders wirksames Austragen der unteren Schichten des Gärstapels G erreicht, wobei durch Verwendung dieses sog. Schub-Zugbodens sowohl eine Brückenbildung und damit ein zu geringer Austrag, als auch ein übermäßiges Durchrutschen von zu viel vergärtem Material sicher vermieden werden.

Nachfolgend wird das in der Anlage verwirktliche Verfahren beschrieben:
1. Befüllung
   Die Biomasse wird mit einer Rohrschnecke durch die Zuführöffnung 1 6 in der Stirnwand 11 bis ca. 30 cm in die Umhausung 1 eingebracht. Dort fällt die Biomasse (zu vergärende Feststoffe) auf den Fußboden 20 des unterflurigen Raumes 2 und wird von einem Kettenförderer 21 Richtung Rückwand 13 entlang der niedrigen Seitenwand 12 weiterbefördert. Der Kettenförderer 21 führt die Biomasse zwischen den Betonsockeln 22, 23 mit seinen Räumschienen 211 in in Fig. 1 dargestellter Transportrichtung X voran. Aufgrund der nur 5 cm hohen Räumschienen 211 des Kettenförderers 21 fällt ein Teil des Fördergutes hinter die jeweilige Räumschiene und wird von der nächsten weiterbewegt. Durch diese Umwälzung und Verteilung während der Förderung entsteht eine ständige Durchmischung der Biomasse. Vor der Rückwand 13 wird die Richtung des Kettenförderers 21 über nicht dargestellte Umlenkrollen bei Mitführung der Masse auf die andere Seite des trapezförmigen Betonsockels 22 entlang der hohen Seitenwand 14 wieder zur Stirnwand zurückbefördert. An der Stirnwand 11 kann wiederum eine Umlenkung der Masse und erneute Kreisförderung durchgeführt werden. Bedarfsweise kann über die im Fußboden 20 eingelassene Förderschnecke 25 die Biomasse der Vorstufe im unterflurigen Raum 2 entnommen werden und einem Vertikalförderer 31 zugeführt werden.
   Bei einer bevorzugten Ausgestaltung werden 7,1 m³ Biomasse eingebracht, was einer durchschnittlichen Befüllhöhe von ca. 30 cm im unterflurigen Raum 2 entspricht. Dieser Vorgang dauert ca. eine halbe Stunde. In dieser Zeit dreht der Kettenförderer 21 mit seinen Räumschienen 211 drei Runden, was einer Geschwindigkeit von ca. 3 m pro Minute ergibt. Nach Abschluss der Befüllung beginnt die Beförderung der Biomasse auf den Gärstapel.
   Die Entleerung des unterflurigen Raumes 2 erfolgt dabei mittels einer Trogschnecke 25, die in einer Rinne nahe der Stirnwand 11 in Förderrichtung des Kettenförderers 21 vor der Zuführöffnung 16 angeordnet ist. Im bevorzugten Ausführungsbeispiel dauert der Vorgang bei der Entleerung ca. 20 Minuten. Die den unterflurigen Raum 2 entleerende Trogschnecke 25 bildet eine funktionelle Einheit in Form einer Zuführschnecke mit einem Vertikalförderer 31 in Form eines Becherelevators, der die vom unterflurigen Raum 2 abgeführte Biomasse nach oben auf einen Bandförderer 32, beispielsweise in Form eines Tragrollenförderbandes fördert. Mit diesem Förderband wird die Biomasse über der flächenmäßigen Mitte des Fermenterraumes 3 aus einer Höhe von ca. 4 m über den Fußboden 20 bzw. 0,8 m über der gewünschten Füllhöhe des Gärstapels G abgeworfen.
   Der dort entstehende Schuttkegel wird mit zwei querliegenden in Längsrichtung bewegbaren Balken 42 als Verteilungsmittel 4 auf dem Gärstapel G zu einer möglichst gleichmäßigen Schicht verteilt. Die beiden über Seilzüge 41 angelenkten Balken 42 sind mit einem Höhenunterschied von ca. 5 cm übereinander angeordnet, wobei der untere Balken 42 auf der angestrebten Füllhöhe des Gärstapels G von ca. 2,45 m über dem Spaltenboden 24 gelagert ist. Der Stahlseilzug 41 wird in gleicher Weise wie ein Miststreuer von einer querliegenden Welle bewegt, die über Zahnräder angetrieben wird. Die Balken 42, beispielsweise in Form von Stahlträgern, bewegen sich aus der Nullstellung (oberer Täger 30 cm vor der hinteren Zwischenwand 18, unterer Träger direkt an der vorderen Zwischenwand 19) gegenläufig zur Mitte und erfassen dort das zu einem Schuttkegel abgeworfene Gut. Da der obere Träger "Vorsprung" hat, erfasst er den oberen Teil des Kegels und bewegt ihn in Richtung Stirnwand 11. Der untere Balken 42 erfasst den unteren Teil des Kegels und befördert ihn Richtung Rückwand 13. Wenn der obere Balken 42 die Vorderwand 19 erreicht hat, schaltet der Antrieb auf Rückbewegung. Während dieser Phase vervollständigt der untere Balken 42 die Arbeit des oberen in der vorderen Hälfte des Fermenterraumes 3. Bei Erreichen der vorderen Zwischenwand 19 schaltet der Antrieb wieder auf Vorwärtsbewegung oder aus. Ein Verteilungszyklus des Verteilungsmittels 4 dauert in einem bevorzugten Ausführungsbeispiel 4 Minuten, wodurch sich eine Arbeitsgeschwindigkeit von 6,5 m pro Minute ergibt. Bei jeder Befüllung wird dieser Vorgang acht mal durchgeführt. Zehn Minuten nach Beginn der Beförderung der Biomasse auf den Gärstapel G wird ein weiterer Befüllungsvorgang für den unterflurigen Raum 2 gestartet. Die Entleerung des unterflurigen Raumes 2 wird nach der letzten Füllung um 10 Minuten verlängert, um auch die letzte hinter die Räumschienen des Kettenförderers 21 gefallene und damit verzögert transportierte Biomasse zu erfassen.
   Zusammengefasst ergibt sich eine Befüllung des Fermenters mit 14,2 m³ Frischmasse. Der Kettenförderer 21 läuft ca. 90 Minuten. Die mittlere Verweildauer der Frischmasse im unterflurigen Raum 2 beträgt ca. 33 Minuten, in der sie von der Wärme der Fußbodenheizung 7 und der Perkolation 5 erfasst wird. Bei diesem Befüllvorgang ergibt sich bei der bevorzugt dimensionierten Ausgestaltung eine Schichtdicke von ca. 33 cm, die annähernd waagerecht auf dem Gärstapel G verteilt ist.
2. Interner Kreislauf
   Dadurch, dass auf der Unterseite des Gärstapels G Schichten mit Biomasse entnommen werden und oben weitere Biomasse zugeführt und zu Schichten verteilt wird, sinkt die betrachtete Schicht im Gärstapel G nach unten (Schichtenströmungsverfahren) und erreicht nach einer bestimmten Zeit den Spaltenboden 24. Dabei wird bei der realen Durchführung ein Teil der vorherigen Schicht auf dem Spaltenboden 24 noch aufliegen, wodurch sich eine erwünschte Mischung zwischen den Schichten ergibt. Die Geschwindigkeit dieses Vorgangs wird durch die Häufigkeit der Entnahmen und der Befüllung gesteuert und im Ausführungsbeispiel so bemessen, dass die betrachtete Schicht nach 7 Tagen auf dem Spalten boden 24 aufliegt. Dabei erfährt sie eine Volumenverkleinerung von angenommen 10 %, weil ein Teil der festen Masse von den Mikroorganismen in Gas umgewandelt wird.
   Auf dem Spaltenboden 24 liegt ein Stahlrahmen aus liegenden T-Trägern, der 17,5 cm kürzer ist, als die Länge des Spaltenbodens 24. Der Stahlrahmen ist in Längsrichtung von drei eingepassten T-Trägern gleichen Maßes verstärkt. Zwischen diesen Längsstreben sind im Abstand von jeweils 16,5 cm auf seiner Unterseite 17,5 cm Breite und ausreichend stabile Stahlplatten untergeschweißt, die die Verschlussbleche 34 bilden. Der Stahlrahmen bildet einen Kratzboden 33, der über nicht dargestellte Hydraulikzylinder um 17,5 cm vor- und zurückbewegbar ist. Dabei sind drei Stellungen der Verschlussbleche 34 auf den Balken 241 des Spaltenbodens 24 vorgesehen, nämlich "auf" (c), "zu" (a) und "1 cm auf" (b), wie dies in Fig. 2 in Vergrößerung dargestellt ist. An dem Stahlrahmen (Kratzboden) sind nach oben ausgerichtete Widerhaken angeordnet, die die unterste Schicht des Gärstapels G auflockern. Bei Betätigung des Kratzbodens 33 in die "auf"-Stellung (c) wird der unterflurige Raum 2 unterhalb des Spaltenbodens 24 fast komplett gefüllt, da eine Steuerung dieses Vorgangs kaum möglich ist. Unmittelbar unter dem Balken 241 bleibt lediglich ein kleiner Raum, in den keine Biomasse rieselt. Im bevorzugten Ausführungsbeispiel ist eine dreimalige Vor- und Zurückbewegung des Kratzbodens 33 ausreichend, um eine fast vollständige Befüllung des unterflurigen Raums 2 zu erreichen. Da das Volumen des unterflurigen Raumes ca. 16 m³ beträgt, wird die vom Gärstapel G abgegebene Menge auf ca. 15,7 m³ abgeschätzt. Somit wird mehr als die betrachtete Schicht (Volumengröße: 14,2 m³ - 10 % = 12,78 m³) in den unterflurigen Raum 2 fallen.
   Alternativ zu dem hier im Verfahrensablauf beschriebenen Kratzboden 33 kann selbstverständlich der zu Fig. 5 beschriebene Schub-Zugboden verwendet werden, der einen sehr präzisen Volumenaustrag bei Vermeidung jeglicher Brückenbildung für das feuchte, schwer handhabbare vergorene Feststoffmaterial liefert. Im Falle der Verwendung eines derartigen Schub-Zugrahmens 35 kann das Austragvolumen so eingestellt werden, dass beispielsweise bei zwei Bewegungszyklen, d. h. einer Zug-, einer Schub-, einer erneuten Zug- und einer erneuten Schubbewegung das gewünschte Volumen von beispielsweise 15,7 m³ ausgetragen wird.
   Vor dem Abfallen der betrachteten Schicht aus dem Gärstapel 6 in den unterflurigen Raum 2 werden somit 3 m³ frische Biomasse in den unterflurigen Raum 2 gefüllt. Somit kann im wesentlichen nur die betrachtete Schicht durch den Spaltenboden 24 nach unten in den unterflurigen Raum 2 augetragen werden, da der Rest des Raumes durch die zugeführte frische Biomasse ausgefüllt ist. Nun wird der Stahlrahmen-Kratzboden 33 auf Stellung "1 cm auf" (b) gestellt, so dass Perkolat aus dem Gärstapel G auf das im unterflurigen Raum 2 befindliche Substrat tropft.
   Dort ruht die Biomasse 30 Minuten um Wärme von der Fußbodenheizung und Perkolat aufzunehmen. Danach wird der Kettenförderer 21, Trogschnecke 25, Becherelevator 31, Tragrollenförderband 32 und der Gärstapelbreitverteiler (Verteilungsmittel) 4 in Bewegung gesetzt und die betrachtete Schicht kommt - wie bereits beim Befüllungsvorgang beschrieben - nun vermischt mit der zugeführten Biomasse erneut auf dem Gärstapel zum liegen. Für die Ablaufzeiten ist dabei für den Vorgang der Befüllung des unterflurigen Raumes mit 3 m³ Frischsubstrat ca. 9 Minuten, für das Betätigen des Kratzbodens 33 ca. 1 Minute und für die Horizontalförderung mit Kettenförderer 21 fünf Runden, entsprechend 50 Minuten zu veranschlagen, so dass sich eine Gesamtdauer von 1 Stunde Aktivität im unterflurigen Raum 2 für den internen Kreislauf ergibt.
3. Entleerung
   Nun sinkt die betrachtete Schicht - wie oben beschrieben - wieder nach unten auf den Spaltenboden 24. In dieser Zeit verliert sie wegen erhöhter bakterieller Aktivität 15 % Volumen, also 14,2 m³- 15 % = 12,07 m³. Auf dem Spaltenboden 24 angekommen wird die Schicht mit Hilfe des Kratzbodens 33 in den unterflurigen Raum 2 befördert. Dort fallen auch ca. 3,6 m³ Biomasse aus der darüber- und/oder darunterliegenden Schicht hinein. Danach läuft der Kettenförderer 10 Minuten und verteilt die Biomasse auch in die Teile des unterflurigen Raumes 2, die nicht unter dem Gärstapel G liegen. Dadurch wird erreicht, dass die Biomasse nicht mehr den Raum bis zum Spaltenboden 24 ausfüllt, sondern sich ein freier Raum in einem Abstand von 5 cm vom Spaltenboden, in den das Biogas entweichen kann.
   Dann ruht die Biomasse eine Stunde um unter der Wärme des Beheizungsmittels 7 (Fußbodenheizung) Biogas zu entwickeln und abzugeben. Nun wird der Kettenförder 21 und die Trogschnecke 25 angestellt, so dass die vergorene Biomasse, da der Becherelevator 31 nicht aktiviert ist, über die Austragöffnung 17 und darin angeordneter Rohrschnecke ausgetragen wird. Die Rohrschnecke befördert die Biomasse aus der Umhausung 1 zur weiteren Verwertung außerhalb der Umhausung 1 heraus.
   Der Entleerungsvorgang dauert im bevorzugten Ausführungsbeispiel 2 Stunden, nämlich 1 Minute Kratzbodenbetätigung, 10 Minuten Kettenförderer, 60 Minuten Ruhe, 40 Minuten Kettenförderer, Trogschnecke und Rohrschnecke, 9 Minuten Nachlaufzeit der Rohrschnecke um den Sammelraum an der Rohrschnecke zu entleeren.
4. Biogasbildung
   Die gasdicht ausgebildete Umhausung 1 bildet im umbauten, nicht belegten Volumen einen Gasspeicher 6, dabei ist das Pultdach 15 beispielsweise kostengünstig mit Trapezprofilen gedeckt, unter denen eine 20 cm starke Dämmung zwischen den Sparren eingefügt ist. Unter die Sparren wird eine Dampfbremsfolie angebracht, Dachlatten aufgeschraubt und innenseitig wiederum Trapezprofile aufgebracht, die mit geeigneten Dichtungsmitteln gasdicht ausgestaltet sind. In der bevorzugt dimensionierten Ausgestaltung beträgt das Gasspeichervolumen 6 ca. 104 m³. Dabei befinden sich ca. 60 m³ oberhalb des Gärstapels G unter dem Pultdach 5, ca. 18 m³ im unterflurigen Raum 2 sowie ca. 17 m³ in den durch Zwischenwänden abgetrennten Seitenräumen. Zwischen dem höchsten Punkt unter dem Pultdach 15 und dem tiefsten Punkt im unterflurigen Raum kurz über dem Fußboden 20 besteht ein Höhenunterschied von 6 m.
   Die Gasentnahme erfolgt dabei über eine erste Gasentnahmeleitung 61 am höchsten Punkt des Pultdaches, bevorzugt an der Rückwand 13 und über eine zweite Gasentnahmeleitung 62 mittig 6 cm über dem Fußboden 20 an der Rückwand 13 im unterflurigen Raum 2. Nur das über Gasentnahmeleitung 61 entnommene Gas wird dem Motor des Blockheizkraftwerks zugeführt. Das an der unteren zweiten Gasentnahmeleitung 62 entnommene Gas wird entweder durch einen Biofilter an die Luft abgegeben oder in einem Tank zur weiteren technischen Verwertung gelagert. Dabei handelt es sich um fast reines CO₂, das sich schwerkraftbedingt im unteren Teil des Gasspeichervolumens 6 sammelt. Um eine möglichst ungestörte schwerkraftbedingte Entmischung des im Gärstapel G erzeugten Biogases zu erreichen, wird die Gasentnahme insbesondere in Zeiten durchgeführt, in denen keine Bewegungen in der Anlage durchgeführt werden. Hierfür stehen mindestens 18 Stunden zusammenhängende Zeit täglich zur Verfügung.
5. Perkolatzirkulation
   Der innerhalb der Umhausung 1 liegende Perkolatflüssigkeitstank 51 hat in bevorzugter Ausgestaltung eine Größe von 3,6 m³. Er besteht bevorzugt aus Edelstahl und ist beispielsweise mit einem Gabelstapler nach Öffnung der Rückwand 13 der Umhausung 1 für Wartungszwecke entnehmbar ausgebildet. Über eine Förderleitung 55 mit Pumpe kann aus dem Perkolatauffangsumpf 54 Perkolat in den Tank 51 rezirkuliert werden. Ferner kann frische Perkolatflüssigkeit über eine Zufuhrleitung 56 in den Perkolatkreislauf 5 eingebracht werden. Aus dem Tank 51 wird über eine Tauchpumpe mit Förderleitung 52 Perkolat zu den Sprühköpfen 53 oberhalb der Gärstapels G gefördert. Dabei wird die Tauchpumpe von einem Schwimmer gesteuert, der bei einem 2,35 m hohen Tank bei 2,3 m Perkolathöhe im Tank anschaltet und bei 0,8 m Füllstandshöhe im Tank abschaltet. Bedarfsweise wird die Perkolatflüssigkeit in dem Tank 51 durch eine darin eingeführte Heizleitung vorgewärmt.
   Die auf dem Gärstapel G versprühte Perkolatflüssigkeit sinkt in dem Gärstapel nach unten und tropft bei "1 cm auf" (b) sowie "auf" (c)-Stellung des Kratzbodens 33 in den unterflurigen Raum 2 ab. Wenn der Kratzboden 33 auf Stellung "zu" (a) steht, staut sich im wesentlichen die Perkolatflüssigkeit in dem Gärstapel auf. Die aus dem Gärstapel tropfende Perkolatflüssigkeit trifft, mit einer Ausnahme, nämlich während der Fermenterbefüllung, in der der Kettenförderer seine erste Runde vollendet hat, auf Biomasse. Das Perkolat wird von der Biomasse mitgeführt. Der gesamte Fußboden 20 des unterflurigen Raumes 2 weist jedoch ein geringes Gefälle zum Auffangsumpf 54 auf, so dass überschüssige Perkolatflüssigkeit in den Auffangsumpf 54 fließen kann. Hier schließt sich der Kreislauf mit der Förderleitung 55, die mittels einer in den Auffangsumpf 54 eingebrachten Tauchpumpe die überschüssige Perkolatflüssigkeit in dem Tank 51 rückbefördert. Bevorzugt ist die Entnahmeleistung der Pumpe im Perkolatflüssigkeitstank 51 größer als die Zuführleistung der Pumpe im Perkolatsumpf 54, um ein Überlaufen des Tanks 51 zu verhindern. Die Häufigkeit und Funktionsfähigkeit der Perkolation kann beispielsweise mit einer Digitalkamera, die auf die Sprühköpfe 53 gerichtet ist, überwacht werden.
6. Zeitliche und mengenmäßige Abstimmung bei einem dimensionsgemäß bevorzugten Ausführungsbeispiel
   Das Volumen des Fermenterraumes 3, der für den Gärstapel G zur Verfügung steht, beträgt ca. 104 m³ (13,3 m x 3,2 m x 2,45 m), wobei als nutzbares Volumen aufgrund von Verteilungsungenauigkeiten des Verteilungsmittels 4 von 100 m³ ausgegangen wird. Bevorzugt gibt es beim Betrieb der Anlage eine Befüllungs- und Entleerungsphase, die 5 Tage dauert, und eine Phase des internen Kreislaufs, die eine Woche dauert. Die Situation vor der Befüllungs- und Entleerungsphase ist so, dass 23 Stunden vorher der interne Kreislauf abgeschlossen wurde. Weil alle Biomasse im Gärstapel schon den internen Kreislauf hinter sich hat, findet in dieser Zeit ein Volumenverlust in Höhe von 15 % ( = 15 m³) in der Woche statt. Umgerechnet auf 23 Stunden sind dies ca. 2 m³. Dann beginnt ein Entleerungsvorgang der zwei Stunden dauert und 15,7 m³ vergorene Biomasse entleert. Danach beginnt ein Befüllungsvorgang der zwei Stunden dauert und 14,2 m³ frische Biomasse einbringt. In dieser Zeit erfährt der Gärstapel durch Biogasbildung einen Volumenverlust von 0,35 m³. Die Differenz zwischen Entleerungs- und Befüllungsmenge beträgt ca. 1,8 m³. Da nun 3,85 m³ Volumen (2 m³ + 0,35 m³ Volumenverlust vor und während der Befüllung und 1,5 m³ Differenz zwischen ausgegebener vergorener Masse und frisch zugeführter Masse) ungenutzt bleiben würden, wird die Befüllungsmenge um diese Zusatzmenge erhöht. Durch diese Zusatzmenge erhöht sich die Befüllungsdauer von den oben angenommenen 90 Minuten auf die hier schon eingerechneten 2 Stunden.
   Diese Berechnungen der Zusatzmenge sind nur beispielhaft, da weitere Einflussfaktoren wie Verdichtung bei unterschiedlicher Substratmischung sowie bakterielle Aktivität etc. eine Rolle spielen. Die Steuerung der Befüllungsmenge geschieht im Beispiel mit Hilfe einer Digitalkamera, auf deren Aufnahmen die aktuelle Befüllhöhe des Gärstapels zu erkennen ist. Während der Befüllung und Entleerung steht der Kratzboden 33 auf Stellung "1 cm auf" (b), während der Ruhephasen auf Stellung "zu" (a).
   Die Entleerung von vergorener Biomasse ist nach 5 Entleerungsvorgängen abgeschlossen. Es standen 100 m³ minus ca. 15 % Volumenverlust (= 85 m³) zur Entleerung bereit. Nach 5 Vorgängen je 15,7 m³ sind 78,5 m³ ausgebracht. Der Rest bleibt als Ausgleichsschicht wegen der Vermischungen zwischen den Schichten im Fermenter. Selbstverständlich wird bei jeder Entleerung auch frisches Material zugeführt, so dass der Fermenter stets mit der gewünschten Befüllhöhe befüllt ist.
   Zur Orientierung ist beispielsweise Freitag. Nach dem Austrag der vergorenen Biomasse und der entsprechenden Neubefüllung ruht der Gärstapel zunächst 68 Stunden. Der Strahlrahmen-Kratzboden 33 wird auf Stellung "zu" (a) gestellt. Montag beginnt der interne Kreislauf. Der Stahlrahmen-Kratzer wird auf Stellung "1 cm auf" (b) gestellt. Im Gärstapel ist ein Volumenverlust von 10 % in der Woche, der auf 68 Stunden umgerechnet ca. 4 m³ ausmacht, entstanden. Es beginnt der interne Kreislauf wie vorangehend beschrieben. Die durch den Volumenverlust benötigte Zusatzmenge wird ab diesem Zeitpunkt während der Beförderung der Biomasse vom unterflurigen Raum 2 auf den Gärstapel G zugeführt. Dadurch verlängert sich die Dauer des internen Kreislaufes auf 2 Stunden. Es folgt eine 22-stündige Ruhephase, in der der Kratzboden 33 auf Stellung "zu" (a) steht. Der interne Kreislauf wird in einer Woche 7 Mal durchgeführt, da jedes mal 1/7 der um 10 % Volumenverlust verringerten Biomasse aus dem Gärstapel entnommen wird, ist dann das gesamte Material vom internen Kreislauf erfasst. Nachfolgend erfolgt wiederum eine Befüllungs- und Entleerungsphase, wie vorangehend beschrieben.
7. Wartung und Reparaturen in der Umhausung
   Vorteilhaft kann der vordere Technikbereich zwischen Stirnwand 11 und Zwischenwand 19, der die wesentlichen technischen Einrichtungen, nämlich Zuführöffnung 16 mit Zuführförderer, Austragöffnung 17 mit Austragförderschnecke, Trogschnecke 25 und Vertikalförderer 31 beinhaltet, gasdicht vom übrigen Raum der Umhausung 1 abgetrennt werden. Damit könnte dieser Raum über eine in der Stirnwand 11 angebrachte gasdichte Tür begehbar sein. Hierzu wird dieser Raumabschnitt mit einer Stahlklappe nach oben gasdicht verschlossen, die zweite Gasentnahmeleitung 62 geöffnet und die Tür in der Stirnwand 11 geöffnet, so dass Umgebungsluft in den Raum fließen kann. Nach Beendigung der Arbeiten wird die Tür verschlossen, die Luft bevorzugt über die zweite Gasentnahmeleitung 62 abgesogen und die Stahlklappe geöffnet.

Bei sonstigen in der Umhausung 1 durchzuführenden Wartungsarbeiten muss der Fermenter 3 entleert werden. Dies kann mittels der beschriebenen Austragtechnik ohne Neubefüllung erfolgen. Sollte die Entleerungstechnik versagen, muss die Rückwand 13 geöffnet und mit Hilfe eines Gabelstaplers zur Seite gestellt werden. Ebenso kann der Perkolatflüssigkeitstank 51 beiseite genommen werden und die bevorzugt aus Holzbohlen erstellte Zwischenwand 18 herausgehoben werden, um den Weg für die maschinelle Entleerung des Gärstapels G mittels Spezialmaschinen aus dem Bauhandwerk zu ermöglichen.

### Bezugszeichenliste

- 1: Umhausung
- 11: Stirnwand
- 12: Seitenwand
- 13: Rückwand
- 14: hohe Seitenwand
- 15: Pultdach
- 16: Zuführöffnung
- 17: Austragöffnung
- 18: Zwischenwand
- 19: Zwischenwand

- 2: unterfluriger Raum
- 20: Fußboden
- 21: Horizontalfördermittel
- 211: Räumschiene
- 22: trapezförmiger Betonsockel
- 23: dreiecksförmiger Betonsockel
- 24: Boden, Spaltenboden
- 241: Balken
- 242: Spalte
- 243: dachförmiger Balken
- 25: Trogschnecke

- 3: Fermenter
- 31: Vertikalförderer
- 32: Bandförderer
- 33: Kratzboden
- 34: Verschlussblech
- 35: Schub-Zugrahmen
- 351: Schubelement
- 36: Hydraulikzylinder

- 4: Verteilungsmittel
- 41: Seilzug
- 42: Balken

- 5: Perkolatzirkulation
- 51: Tank
- 52: Förderleitung
- 53: Sprühkopf
- 54: Auffangsumpf
- 55: Förderleitung
- 56: Zuführleitung

- 6: Gasspeichervolumen
- 61: erste Gasentnahmeleitung
- 62: zweite Gasentnahmeleitung

- 7: Beheizungsmittel
- 71: Heizrohr

- G: Gärstapel
- X: Transportrichtung

## Patentansprüche

1. Verfahren zur Biogaserzeugung durch Trockenfermentation, bei dem zu vergärender Feststoff einem Fermenter zugeführt, ggf. mit Perkolatflüssigkeit benetzt und unter Bildung von Biogas (Methan, Kohlendioxid) vergärt wird, **gekennzeichnet durch** die Schritte:
- Durchmischen und Erwärmen der zugeführten zu vergärenden Feststoffe in einer Vorstufe,
- Verteilen der erwärmten und durchmischten Feststoffe aus der Vorstufe auf dem im Fermenter befindlichen Gärstapel,
- Austragen von vergärten Feststoffen auf der Unterseite des im Fermenter befindlichen Gärstapels,
wobei die zugeführten, zu vergärenden Feststoffe in der Vorstufe auf einer im wesentlichen horizontalen Förderbahn unterhalb des Gärstapels geführt werden, während Perkolatsflüssigkeit und/oder kleinere Fraktionen aus dem Gärstapel auf die zu vergärenden Feststoffe niedertropfen oder -fallen.

2. Verfahren nach Anspruch 1, **dadurch gekennzeichnet, dass** die Förderbahn einen Förderkreislauf bildet, der von den zu vergärenden Feststoffen wenigstens einmal durchlaufen wird, bevor die dann durchmischten Feststoffe zur Verteilung auf dem Gärstapel emporgefördert werden.

3. Verfahren nach Anspruch 1 oder 2, **dadurch gekennzeichnet, dass** zum Austragen der vergorenen Feststoffe die Zufuhr von zu vergärenden Feststoffen gestoppt wird und vergorene Feststoffe aus dem Gärstapel auf die darunterliegende Förderbahn abgegeben und von dort ausgetragen werden.

4. Verfahren nach Anspruch 3, **dadurch gekennzeichnet, dass** die auszugebenden vergorenen Feststoffe den Förderkreislauf der Förderbahn wenigstens einmal durchlaufen.

5. Verfahren nach einem der vorangehenden Ansprüche, **dadurch gekennzeichnet, dass** teilweise vergorene Feststoffe auf die Förderbahn abgegeben, dort mit frisch zugeführten, zu vergärenden Feststoffen gemischt und wieder auf dem im Fermenter befindlichen Gärstapel verteilt werden (interner Kreislauf).

6. Verfahren nach Anspruch 5, **dadurch gekennzeichnet, dass** bei einem vollständig befüllten Fermenter nach dem internen Kreislauf zunächst eine Teilmenge des Gärstapels über die Förderbahn ausgegeben wird, dann zu vergärende Feststoffe über die Förderbahn zugeführt und auf dem Gärstapel verteilt werden und anschließend wieder ein interner Kreislauf durchgeführt wird.

7. Verfahren nach einem der vorangehenden Ansprüche, **dadurch gekennzeichnet, dass** das Biogas beim Auffangen schwerkraftbedingt im wesentlichen in eine Methanfraktion und eine Fraktion der übrigen Bestandteile, insbesondere Kohlendioxid getrennt wird.

8. Anlage zur Biogaserzeugung durch Trockenfermentation mit einer im wesentlichen gasdichten Umhausung (1), in der ein Fermenter (3) ggf. mit Perkolatflüssigkeitskreislauf (5) angeordnet ist, dem über ein Zufuhrmittel (16) zu vergärende Feststoffe zuführbar und über ein Austragmittel (17) vergorene Feststoffe austragbar sind, **dadurch gekennzeichnet,**
- **dass** der Fermenter (3) einen mit Öffnungen versehenen oder öffenbaren Boden (24) aufweist,
- **dass** unter dem Boden (24) des Fermenters (3) ein unterfluriger Raum (2) in der gasdichten Umhausung (1) vorgesehen ist,
wobei im unterflurigen Raum (2) Horizontalfördermittel (21) und Beheizungsmittel (7) angeordnet sind, um die zugeführten, zu vergärenden Feststoffe zu fördern, zu durchmischen und zu erwärmen,
- und **dass** ein Vertikalförderer (31) vorgesehen ist, der die durchmischten, erwärmten, zu vergärenden Feststoffe auf einen im Fermenter (3) befindlichen Gärstapel (G) ablegt.

9. Anlage nach Anspruch 8, **dadurch gekennzeichnet, dass** der Boden (24) des Fermenters (3) Spalten aufweist, insbesondere ein Spaltenboden (24) ist, wobei die Spalten (242) bevorzugt verschließbar oder von dachförmigen Balken (243) überdeckt ausgebildet sind.

10. Anlage nach Anspruch 9 mit einem Spaltenboden (24), **dadurch gekennzeichnet, dass** ein bevorzugt hydraulisch hin- und herbewegbarer Kratzboden (33) mit Verschlussblechen (34) oder Schub-Zugrahmen (35) mit leiterförmigen Schubelementen (351) auf dem Boden (24) aufliegt.

## Claims

1. A process for generating biogas by dry fermentation, wherein solid matter to be fermented is supplied to a fermenter, optionally wetted with percolating liquid and, under formation of biogas (methane, carbon dioxide), fermented, **characterised by** the following steps:
- in a preliminary stage, mixing and heating of the supplied solids to be fermented;
- distribution of the heated and mixed solids from the preliminary stage over the fermentation batch contained in the fermenter;
- discharge of fermented solids from the underside of the fermentation batch contained in the fermenter;
wherein, in the preliminary stage, the supplied solids to be fermented are transported on a substantially horizontal conveyor underneath the fermentation batch, while percolating liquid and/or smaller fractions from the fermentation batch drip or fall down onto the solids to be fermented.

2. A process according to claim 1, **characterised in that** the conveyor forms a conveying circuit, through which the solids to be fermented pass at least once before the solids then mixed are conveyed upwards for distribution over the fermentation batch.

3. A process according to claim 1 or 2, **characterised in that**, to discharge the fermented solids, the supply of solids to be fermented is stopped and fermented solids are transferred from the fermentation batch to the underlying conveyor and from there are discharged.

4. A process according to claim 3, **characterised in that** the fermented solids to be transferred pass through the conveying circuit of the conveyor at least once.

5. A process according to any one of the preceding claims, **characterised in that** partially fermented solids are transferred to the conveyor, are mixed there with freshly supplied solids to be fermented and are again distributed over the fermentation batch contained in the fermenter (internal circuit).

6. A process according to claim 5, **characterised in that**, in the case of a completely filled fermenter after the internal circuit, first a partial quantity of the fermentation batch is transferred via the conveyor, then solids to be fermented are supplied via the conveyor and distributed over the fermentation batch, and subsequently an internal circuit is again executed.

7. A process according to any one of the preceding claims, **characterised in that** the biogas, when collected, is separated by gravity substantially into a methane fraction and a fraction comprising the remaining constituents, especially carbon dioxide.

8. An installation for the generation of biogas by dry fermentation, with a substantially gas-tight housing (1) in which, optionally with a percolating-liquid circuit (5), a fermenter (3) is arranged, to which solids to be fermented can be supplied via a supply means (16) and from which fermented solids can be discharged via a discharge means (17), **characterised in that**
- the fermenter (3) has a floor (24) which is openable or is provided with openings,
- **in that** an underfloor space (2) is provided under the floor (24) of the fermenter (3) in the gas-tight housing (1),
wherein horizontal conveying means (21) and heating means (7) are arranged in the underfloor space (2) in order to convey, mix and heat the supplied solids to be fermented,
- and **in that** a vertical conveyor (31) is provided which deposits the mixed and heated solids to be fermented onto a fermentation batch (G) contained in the fermenter (3).

9. An installation according to claim 8, **characterised in that** the floor (24) of the fermenter (3) has gaps, in particular is a slatted floor (24), wherein the gaps (242) are preferably closable or are formed so as to be covered by roof-shaped beams (243).

10. An installation according to claim 9 with a slatted floor (24), **characterised in that** a scraper floor (33) with closing plates (34), which is preferably hydraulically movable back and forth, or a push-pull frame (35) with ladder-type pushing members (351) rests on the floor (24).

## Revendications

1. Procédé pour la production de biogaz par fermentation sèche, dans lequel la matière solide devant être fermentée est délivrée à un fermenteur, le cas échéant humectée avec un liquide de percolation et fermentée en formant du biogaz (méthane, dioxyde de carbone), **caractérisé par** les étapes consistant à :
- malaxer et chauffer les matières solides délivrées devant être fermentées, lors d'une étape préalable,
- répartir les matières solides chauffées et malaxées, issues de l'étape préalable, sur la pile de fermentation présente dans le fermenteur,
- évacuer les matières solides fermentées sur la face inférieure de la pile de fermentation présente dans le fermenteur,
les matières solides délivrées et devant être fermentées étant guidées lors de l'étape préalable sur un convoyeur sensiblement horizontal en dessous de la pile de fermentation, tandis que le liquide de percolation et/ou des fractions plus petites issues de la pile de fermentation tombent goutte à goutte ou petit à petit sur les matières solides devant être fermentées.

2. Procédé selon la revendication 1, **caractérisé en ce que** le convoyeur forme un cycle de transport qui est parcouru par les matières solides devant être fermentées au moins une fois avant que les matières solides alors mélangées ne soient transportées vers le haut à des fins de répartition sur la pile de fermentation.

3. Procédé selon la revendication 1 ou 2, **caractérisé en ce que**, afin d'évacuer les matières solides fermentées, l'alimentation en matières solides devant être fermentées est arrêtée, et les matières solides fermentées sont déchargées de la pile de fermentation pour parvenir sur le convoyeur qui se trouve en dessous, et sont alors évacuées de cet endroit.

4. Procédé selon la revendication 3, **caractérisé en ce que** les matières solides fermentées devant être évacuées parcourent le cycle de transport du convoyeur au moins une fois.

5. Procédé selon l'une des revendications précédentes, **caractérisé en ce que** des matières solides partiellement fermentées sont déchargées sur le convoyeur, et mélangées à cet endroit avec les matières solides qui viennent d'être délivrées et qui doivent être fermentées, puis de nouveau réparties sur la pile de fermentation présente dans le fermenteur (cycle interne).

6. Procédé selon la revendication 5, **caractérisé en ce que**, dans un fermenteur rempli intégralement, après le cycle interne, tout d'abord une quantité partielle de la pile de fermentation est évacuée par l'intermédiaire du convoyeur, puis les matières solides devant être fermentées sont délivrées par l'intermédiaire du convoyeur et réparties sur la pile de fermentation, puis un cycle interne est à nouveau réalisé.

7. Procédé selon l'une des revendications précédentes, **caractérisé en ce que** le biogaz, lorsqu'il est recueilli, est séparé sous l'effet de la force de gravité essentiellement en une fraction de méthane et en une fraction de composant résiduel, en particulier du dioxyde de carbone.

8. Installation pour la production de biogaz par fermentation à sec avec une enveloppe (1) sensiblement étanche au gaz dans laquelle est disposé un fermenteur (3), le cas échéant avec un cycle de liquide de percolation (5), auquel peuvent être délivrées des matières solides devant être fermentées par l'intermédiaire d'un moyen d'alimentation (16), et duquel les matières solides fermentées peuvent être évacuées par l'intermédiaire d'un moyen d'évacuation (17), **caractérisée:**
- **en ce que** le fermenteur (3) présente un fond (24) doté d'ouvertures ou pouvant être ouvert,
- **en ce qu'**il est prévu sous le fond (24) du fermenteur (3) un espace souterrain (2) dans l'enveloppe (1) étanche au gaz,
des moyens de transport horizontaux (21) et des moyens de chauffage (7) étant disposés dans l'espace souterrain (2) afin de transporter les matières solides délivrées devant être fermentées, de les malaxer et de les réchauffer,
- et **en ce qu'**il est prévu un convoyeur vertical (31) qui dépose les matières solides malaxées, réchauffées et devant être fermentées sur une pile de fermentation (G) présente dans le fermenteur (3).

9. Installation selon la revendication 8, **caractérisée en ce que** le fond (24) du fermenteur (3) présente des interstices, en particulier un caillebotis (24), les interstices (242) étant de préférence configurés de manière à pouvoir être fermés ou en étant recouverts par des poutres (243) en forme de toit.

10. Installation selon la revendication 9 comportant un caillebotis (24), **caractérisée en ce qu'**un fond mouvant (33), pouvant effectuer des mouvements de va-et-vient de préférence par voie hydraulique, avec des tôles de fermeture (34) ou un châssis de poussée/traction (35) comportant des éléments de poussée (351) en forme d'échelles, repose sur le sol (24).
